# EUROPEAN PATENT APPLICATION

(11) **EP 3 632 924 A1**
(43) Date of publication of application: **08.04.2020**
(21) Application number: 18306324.7
(22) Date of filing: 07.10.2018
(51) Int. Cl.: C07K 14/195

(54) **BINDERS FOR INHIBITING FORMATION OF MULTIMERIC PROTEINS**

(71) Applicant: AFFILOGIC, 44200 Nantes (FR)
(72) Inventor: The designation of the inventor has not yet been filed
(74) Representative: Flesselles, Bruno F.G.

(57) **Abstract**

The invention relates to variants of OB-fold proteins, in particular of the Sac7d family that are able to bind a subunit of a multimeric protein and inhibit the formation of the multimer.

## Description

The invention is in the field of molecular biology and relates in particular to the development of novel molecules and conjugates having the ability to inhibit the formation of multimeric protein complexes.

### Introduction

Some proteins having biological properties may be active as multimeric proteins, i.e. polypeptides that are formed by multiple polypeptide chains, called subunits. A multimeric protein or polypeptide may be formed of the same subunits (homomultimer) or of different subunits (heteromultimer). Consequently, a heteromultimeric polypeptide describes a protein containing two or more different polypeptide chains, whereas a homomultimeric polypeptide describing a protein containing two or more polypeptide chains that are all identical.

Generally, *in vivo,* there is an equilibrium between the monomeric subunits and the multimeric protein in soluble form, with monomeric subunit getting assembled to form the multimeric protein and multimeric protein dissociating and releasing the multimeric forms. The ratio between the different species is governed by a concentration based equilibrium that shift toward the multimeric form at relatively high concentration. Such equilibrium allows regulating the concentration of the multimeric and active soluble form to maintain homeostasis.

In order to reduce the biological activity of multimeric proteins, and in particular of their soluble form over their membrane-bound form which is stabilized when inserted in the cell membrane, one strategy is to avoid the formation of the biologically active multimeric protein or shift the equilibrium towards the monomeric (or multimeric inactive) subunits. This may be performed by blocking the assembly of the subunit, for instance by sterically blocking the site of assembly of the multimer.

It is thus interesting to develop molecules that would bind to a monomeric part of a multimeric protein and inhibit the formation of the multimer, or move the monomeric-multimeric species equilibrium.

The applicant proposed to produce such molecules by creating novel artificial proteins that are able to bind a sub-unit of a multimeric protein and to inhibit the formation of the multimeric complex, based on variants of an OB-fold domain that can be, in particular, obtained by a specific method of screening.

### SUMMARY OF THE INVENTION

The invention relates to variants of a OB-fold protein, in particular a protein of the Sac7d family that inhibit the formation of a biologically active multimeric protein, by binding a subunit protein at an epitope involved in such formation of the multimer, or to a sub-multimer, thereby preventing the formation of the biologically active multimer.

The invention also encompasses a polypeptide comprising such variant of a protein of the Sac7d family.

It is to be noted that the variant comprises between 4 and 22 mutated amino acids in the binding site of the protein of the Sac7d family, and binds to a subunit of a multimeric protein but doesn't bind to the subunit of the protein when such subunit protein is involved in the fully formed multimeric protein in its native state.

It is to be noted that the specification discloses below the Sac7d proteins and family, but the teachings are applicable to other OB-fold domains as disclosed in WO2007139397. The invention is also applicable to SH3 domains, a small protein domain of about 60 amino acid residues, initially, described as a conserved sequence in the viral adaptor protein v-Crk and described under PF00018 in the PFAM database. The SH3 domain has a characteristic beta-barrel fold that consists of five or six β-strands arranged as two tightly packed anti-parallel β sheets. The linker regions may contain short helices. It is to be noted that OB-fold and SH3 domains share homology and that, in view of the knowledge of the sequence and structure of these domains, it is possible to determine, in any of OB-fold or SH3 domain, to which amino acids correspond the amino acids as disclosed below for Sac7d.

In specific embodiments, the multimeric protein is part of the TNF-alpha superfamily, and more specifically TNF-alpha, RANKL or TRAIL (TNFSF10).

In specific embodiments, the variant is present in a polypeptide and is thus covalently linked by amine bounds to other proteins presenting a biological interest.

In specific embodiments, the variant is conjugated to an organic molecule that presents some functionality.

The invention also relates to these variants in therapeutic, diagnostic or purification uses. The invention also relates to composition, in particular oral or topical (dermal), containing the variants.

The invention also pertains to a method that makes it possible to identify such variants that bind to the face of subunits that are involved in the formation of multimeric proteins (faces that are involved in the protein-protein interactions in the multimer.

According to the invention, it is possible to obtain a polypeptide comprising a variant of a protein of the Sac7d family, the variant comprising between 4 and 22 mutated amino acids in the binding site of the protein of the Sac7d family, that binds to a subunit of a multimeric protein and that doesn't bind to the subunit of the protein when such subunit protein is involved in the fully formed multimeric protein in its native state. It is reminded that the sequence of Sac7d is:

MVKVKFKYKGEEKEVDTSKIKKVWRVGKMVSFTYDDNGKTGRGAVSEKDAPKEL
LDMLARAEREKK (SEQ ID NO: 1).

It is possible to obtain the above polypeptide, wherein the mutated amino acids of the Sac7d variant are selected from the group consisting of V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51 of Sac7d.

It is possible to obtain the above polypeptide, wherein the Sac7d variant contains 4 to 17 mutated amino acids selected from the group corresponding to K7, Y8, K9, E11, K21, K22, W24, V26, M29, S31, T33, D35, T40, G41, R42, A44, and S46 of Sac7d.

In a preferred embodiment, the multimeric protein is part of the Tumor necrosis factor superfamily, and is preferably TNF-alpha, RANKL or TRAIL.

In preferred embodiments, the polypeptide comprises the sequence 
MVKVKFXXXGXEKEVDTSKIXXVXRXGKXVHFWYXDNGKIXKGXVXEKDAPKELL
DMLARAEREK (SEQ ID NO: 17)
 
MVKVKFXXXGXEKEVDTSKIXHVMRXGKXVHFWYEDNGKIXKGXVXEKDAPKEL
LDMLARAEREK (SEQ ID NO: 18)
 or 
MVKVKFVMFGKEKEVDTSKIVHVMRQGKLVHFWYXDNGKIDKGFVPEKDAPKEL
LDMLARAEREK (SEQ ID NO:16).
 The polypeptide may also comprise SEQ ID NO: 16, where between 1 to 13, preferably from 1 to 11, more preferably from 1 to 10, more preferably from 1 to 8, more preferably from 1 to 6, more preferably from 1 to 5, more preferably from 1 to 4, more preferably from 1 to 3, more preferably 2, more preferably 1 amino acid selected from the group consisting of V7, M8, F9, K11, V21, H22, M24, Q26, L29, E35, D41, F44 and P46, more preferably from the group consisting of V7, M8, F9, K11, V21, Q26, L29, E35, D41, F44 and P46, have been replaced by another amino acid.

In some embodiments, the polypeptide is such that the variant of a protein of the Sac7d family binding to the subunit of a multimeric protein is linked or fused to another protein or polypeptide, in particular another variant of a protein of the Sac7d family or an antibody, in particular binding to IL17, TNF-alpha or Her2/neu.

In another embodiment, the polypeptide is conjugated to an organic molecule.

It also allows obtaining a genetic construct comprising a DNA sequence coding for a polypeptide defined above, a vector comprising such genetic construct and a host cell comprising such genetic construct in its genome.

It is possible to perform a method for producing the polypeptide, comprising the steps consisting of culturing a cell culture wherein the cells have been transformed by a genetic construct, and recovering the polypeptide.

A method for obtaining and identifying a protein that binds to a monomer of a multimeric protein is also available.

One can also embody a polypeptide as disclosed as a medicament, a polypeptide as disclosed for use thereof for the inhibition of the multimerization of a multimeric protein or for treating diseases as indicated below.

Also encompassed is a method for detecting the presence of, or for quantifying, a subunit of a multimeric protein in a sample, comprising the step of
a. Exposing the sample to a variant that binds to the subunit, in such conditions that binding is possible
b. Recovering the variant and/or detecting or measuring the amount of, the subunit.

Also encompassed is a method for purifying subunit of a multimeric protein in a sample, comprising the step of
a. Exposing the sample to a variant as disclosed herein, that binds to the subunit, in such conditions that binding is possible
b. Recovering the variant bound to the subunit
c. Eluting the subunit from the variant.

### DETAILED DESCRIPTION OF THE INVENTION

### The Sac7d protein family

The Sac7d family is defined as relating to the Sac7d protein and corresponds to a family of 7 kDa DNA-binding proteins isolated from extremophilic bacteria. It is herein disclosed as a representative species of OB-fold domains, that is preferably used in the context of the invention. Since SH3 domains share homology with OB-fold domains, the teachings pertaining to Sac7d are also applicable to SH3 scaffolds.

These proteins and this family are in particular described in WO 2008/068637. Thus, within the context of the present invention, a protein belongs to the Sac7d family when it has one of the sequences SEQ ID NO: 1 to SEQ ID NO: 14, or when it has a sequence corresponding to the sequence SEQ ID NO: 15, which is a consensus sequence (obtained from SEQ ID NO: 1 to SEQ ID NO: 9 and SEQ ID NO: 12 to SEQ ID NO: 14. In this consensus sequence, a dash - indicates no amino acid, the proteins not having all the same size). This Sac7d family comprises in particular the Sac7d or Sac7e proteins derived from *Sulfolobus acidocaldarius,* the Sso7d protein derived from *Sulfolobus solfataricus,* the DBP 7 also called Sto7 protein derived from *Sulfolobus tokodaii,* the Ssh7b protein derived from *Sulfolobus shibatae,* the Ssh7a protein derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and the p7ss protein derived from *Sulfolobus solfataricus.* In view of the broad similarity of the sequences of the proteins of the Sac7d family, it is directly possible and easy to identify the amino acids of another protein than Sac7d that correspond to given amino acids of Sac7d. In particular, one can also use the teachings of WO 2008/068637 that shows (in the figures) and explains (in the specification) that such proteins are superimposable.

It is to be noted that the number of mutated residues in said variant (relative to the wild-type protein) is preferably between 4 and 25, or more specifically between 4 and 22. The invention can be implemented with variants preferably having at least 4, more preferably at least 5, more preferably at least 6, more preferably at least 7 or 8, even more preferably at least 10, but generally less than 25, more preferably less than 22, even more preferably less than 20 or less than 15 or 14 substituted amino acids compared with the wild-type OB-fold protein (or domain). It is to be noted that all and any ranges are considered herein, (such as 5-20 or 7-25 and so forth). Particularly preferred ranges are 4-17 and 6-17, 4-14 and 6-14.

It is preferred when 7, 8, 9, 10, 11, 12, 13 or 14 amino acids are mutated in the binding site of the OB-fold domain, relative to the wild-type OB-fold domain. These mutations are introduced in the amino acids corresponding to V2, K3, K5, K7, Y8, K9, G10, E11, K13, E14, T17, K21, K22, W24, V26, G27, K28, M29, S31, T33, Y34, D36, N37, G38, K39, T40, R42, A44, S46, E47, K48, D49, A50 and P51 of Sac7d (SEQ ID NO: 1)

In a particular embodiment, the number of mutated amino acids is between 7 and 14 (limits included).

In one particular embodiment, these variants can also comprise amino acid insertions as indicated above.

As indicated, proteins of the Sac7d family are Sac7d or Sac7e derived from *Sulfolobus acidocaldarius,* Sso7d derived from *Sulfolobus solfataricus,* DBP 7 also called Sto7 derived from *Sulfolobus tokodaii,* Ssh7b derived from *Sulfolobus shibatae,* Ssh7a derived from *Sulfolobus shibatae,* Mse7 derived from *Metallosphaera sedula,* Mcu7 derived from *Metallosphaera cuprina,* Aho7a or Aho7b or Aho7c derived from *Acidianus hospitalis,* Sis7a or Sis7b derived from *Sulfolobus islandicus* and p7ss derived from *Sulfolobus solfataricus.* The various sequences of the Sac7d, Sso7d, Sac7e, Ssh7b, Ssh7a, DBP7, Sis7a (3 alleles), Mse7, Mcu7, Aho7a, Aho7b and Aho7c proteins are represented by SEQ ID NO: 1 to SEQ ID NO: 14 respectively.

A variant of a protein of this Sac7d family may be called a nanofitin. The invention is thus preferentially implemented on variants of the proteins represented by SEQ ID NO: 1 to SEQ ID NO: 14, or a protein having the sequence SEQ ID NO: 15, in particular on variants of Sac7d.

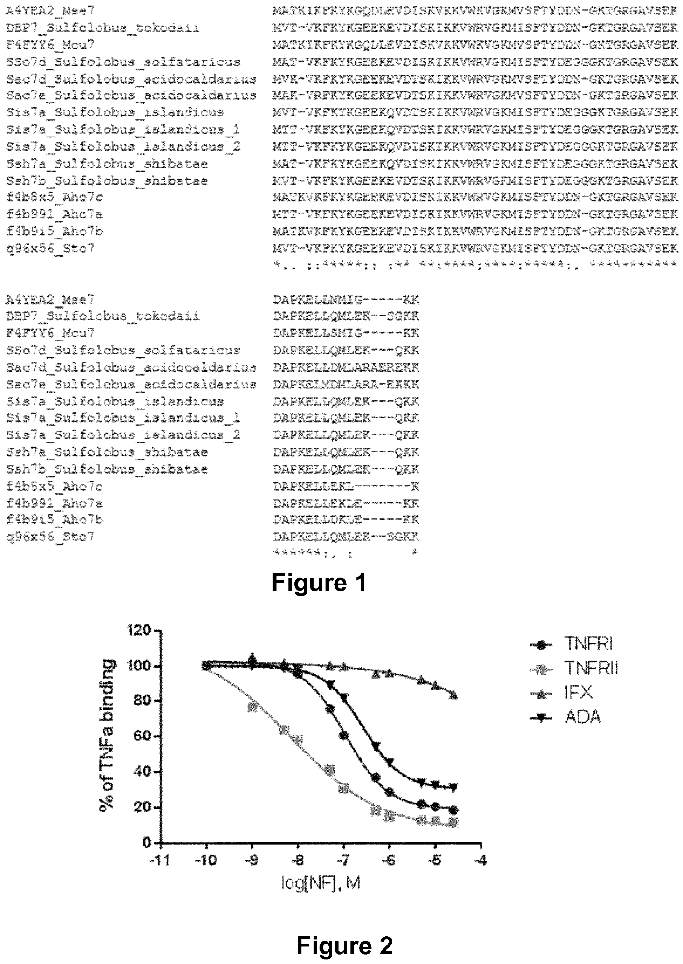

In a preferred embodiment, the mutated amino acids are selected in the group consisting of V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51 (reference is made to SEQ ID NO: 1, and to the alignment of **Figure 1**). It is to be noted that the variant shall comprise mutated amino-acids (preferably from 7 to 12 as indicated above) chosen among these amino acids, and may also contain other mutated amino acids (preferably from 0 to 5) in other regions (i.e. chosen among other residues of Sa7d). As indicated above, A59, R60, A61, E62, R63, E64 and/or K66 can be deleted.

In a preferred embodiment, the mutated amino acids are selected from the group consisting of K7, Y8, K9, E11, K21, K22, W24, V26, M29, S31, T33, D35, T40, G41, R42, A44, and S46.

The variants herein disclosed can be obtained by the method described in WO 2008/068637 (various rounds of enrichment of the combinatorial libraries, in particular using ribosome display).

The variant of OB-fold domain is thus preferably a variant of a protein of the Sac7d family (Sac7d variant), comprising from 5 to 20 (preferably from 7 to 14) mutated amino acids in the binding site of the protein. Preferably, the mutated amino acids of the Sac7d variant are selected from the group consisting of V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51 (in reference to SEQ ID NO:1 and the alignment of Figure 1). Preferably the Sac7d variant contains 4 to 17 mutated amino acids chosen in the group consisting of K7, Y8, K9, E11, K21, K22, W24, V26, M29, S31, T33, D35, T40, G41, R42, A44, and S46.

The advantage of the method described in WO 2008/068637 is that it makes it possible to obtain variants of OB-fold proteins by screening combinatorial libraries containing or expressing a plurality of variants in which a certain number of amino acids have been "randomized", i.e. replaced with a random amino acid. The screening of these libraries makes it possible to identify variants of these proteins which bind specifically, generally with a strong affinity (application WO 2008/068637 in fact describes affinities of the order of one nanomolar), with a target of interest, other than the natural ligand of the wild-type protein from which the combinatorial library was generated. Below are described specific methods to identify variants that bind to the subunit of a multimeric protein so as to prevent formation of the multimer.

### Binding site of the Sac7d protein

The OB-fold proteins are known in the art. They are in particular described in the documents cited above, and also in Arcus (Curr Opin Struct Biol. 2002 Dec; 12(6):794-801). OB-fold is in the form of a cylinder having five beta (β) sheets. Most OB-fold proteins use the same binding interface of their natural ligand, which may be an oligosaccharide, an oligonucleotide, a protein, a metal ion or a catalytic substrate. This binding interface comprises mainly the residues located in the beta sheets. Certain residues located in the loops may also be involved in the binding of an OB-fold protein with its natural ligand. Thus, applications WO 2007/139397 and WO 2008/068637 and the Arcus document (2002, op. cit.) describe the OB-fold-protein domains for binding with their natural ligand.

In particular, document WO 2008/068637 describes precisely how to identify the binding domain of an OB-fold protein. By superimposing several sequences and 3D structures of proteins having OB-fold domains, using the websites WU-Blast2 (http://www.ebi.ac.uk/blast2/index.html) (Lopez et al., 2003, Nucleic Acids Res 31, 3795-3798), T-COFFEE (http://www.ch.embnet.org/software/TCoffee.html) (Notredame et al., 2000, J Mol Biol 302, 205-217) and DALI lite (http://www.ebi.ac.uk/DaliLite/) (Holm and Park, 2000, Bioinformatics 16, 566-567), it is possible to identify the positions of the binding domains and in particular the amino acids which can be modified. Taking the sequence of Sac7d (SEQ ID NO: 1) as a reference, these are the residues V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51.

The binding domains of other OB-fold proteins can be identified as described in WO 2008/068637. This application indicates that it is possible to perform a superimposition of 3D structures of OB-fold proteins or domains (10 domains were used in this application, including Sac7d), using the DALI website (http://www.ebi.ac.uk/dali/interactive.html)(Holm and Sander, 1998, Nucleic Acids Res 26, 316-319). Thus, it is easy to identify, for any OB-fold protein (or any OB-fold domain), the amino acids involved in the binding site and corresponding to the Sac7d amino acids mentioned above. Consequently, providing the amino acids that can be mutated in one of these proteins makes it possible to identify the corresponding amino acids for any other OB-fold domain.

It is also possible to delete some amino acids from the OB-fold scaffold. Still with this Sac7d sequence as reference, the residues which can be deleted are: A59, R60, A61, E62, R63, E64 and/or K66.

The teaching of WO 2008/068637 also indicates that amino acids can optionally be inserted into the loops of the OB-fold proteins, in particular the proteins of the Sac7d family; in particular, insertions of 1 to 15 amino acid residues can be made in loop 3 (as defined in figures 1b and 2 of WO 2008/068637), for example in the region of residues 25 to 30 of Sac7d, preferably between residues 27 and 28, insertions of 1 to 15 amino acid residues can be made in loop 4 (as defined in figures 1b and 2 of WO 2008/068637), for example in the region of residues 35-40 of Sac7d, preferably between residues 37 and 38, and insertions of 1 to 20 residues can be made in loop 1 (as defined in figures 1b and 2 of WO 2008/068637), for example in the region of residues 7 to 12 of Sac7d, preferably between residues 9 and 10.

### Obtaining mutants of an OB fold, in particular of a Sac7d protein

WO 2007/139397 describes the use of libraries of OB-fold proteins in which the OB domain is modified by introducing mutations into this domain for binding of the protein to its natural ligand. In particular and as foreseen herein, modified OB-fold domain comprises a) at least one modified amino acid residue in a β-strand of the OB-fold domain binding face as compared to the naturally occurring OB-fold domain, or b) at least one modified amino acid residue in a β-strand of the OB-fold domain binding face and at least one modified amino acid residue in a strand of the OB-fold domain loop region, or c) at least one modified amino acid residue in a strand of the OB-fold domain loop region. Generally, the modified OB-fold domain has altered binding characteristics as compared to the naturally occurring OB-fold domain.

WO 2008/068637 describes the use of a library based on the Sac7d protein for obtaining ligands which have an affinity for targets of interest. The method described in WO 2008/068637 comprises the generation of combinatorial libraries containing a plurality of DNA molecules all having the same sequence, except for the presence of certain random mutations leading to the production of variants of a wild-type protein, exhibiting mutations at certain amino acids of the binding site of this wild-type OB-fold protein. In particular, in the context of WO 2008/068637, the wild-type OB-fold protein is a Sac7d protein, into which mutations are introduced in order to generate a variability, in particular at amino acids chosen from K7, Y8, K9, E11, K21, K22, W24, V26, M29, S31, T33, D35, T40, G41, R42, A44, and S46, or on other amino acids, such as V26, G27, K28, M29, S31, R42, A44, S46, E47 and K48. These amino acids are based on the Sac7d sequence, as represented by SEQ ID NO: 1.

WO 2012/150314 shows that the mutations from one protein of the Sac7d family can be carried to another protein of the same family. This portability amounts to creating a mutant of another protein of the Sac7d family, starting from a mutant of one protein of said family. The first mutant may have been obtained in particular by carrying out the process of WO 2008/068637. Consequently, it is possible, using in particular the teachings of WO 2012/150314 and of figure 1, to obtain a mutant of any protein of the Sac7d family, starting from a mutant of any other protein of such family. As an illustration, for a mutant of Sac7d, one only has to introduce the mutated amino acids of the Sac7d mutant within the scaffold of another protein, using the sequence alignment of figure 1.

It has already been shown that it is possible to obtain such mutants against a given target (and especially when the target is a protein or a peptide). One can cite variants binding to immunoglobulins (Behar et al, Protein Engineering, Design & Selection vol. 26 no. 4 pp. 267-275, 2013), or to other proteins (WO WO 2008/068637). Gera et al (J Mol Biol. 2011 Jun 17;409(4):601-16) have also shown the possibility to obtain such proteins starting from Sso7d. Gocha et al (Scientific Reports 7, Article number: 12021 (2017)) also reported ability to obtain mutants from Sso7d.

### Example of OB-fold or SH3 domain

Non-limitative examples of OB-fold proteins which can be used according to the invention are Sac7d, Sso7d, the N-terminal domain of SEB (Papageorgiou et al., 1998), the chain A of the Shiga-like toxin IIe (PDB 2bosa), the human Neutrophil Activatin Peptide-2 (NAP-2, PDB 1tvxA), the Molybdenum Binding Protein (modg) of Azotobacter vinelandii (PDB 1h9j), the N-terminal domain of SPE-C (Roussel et al., 1997), the B5 subunit of E. coli Shiga-like toxin (Kitov et al., 2000), Cdc13 (Mitton-Fry et al., 2002), the cold-shock DNA-binding domain of the human Y-box protein YB-1 (Kloks et al., 2002), the E. coli inorganic pyrophosphatase EPPase (Samygina et al., 2001), or any of the proteins listed in Table 3 of the article by (Arcus, 2002), such as 1krs (Lysyl-tRNA synthetase LysS, E.coli), 1c0aA (Asp- tRNA synthetase, E.coli), 1b8aA (Asp- tRNA synthetase, P. kodakaraensis), 1lylA (Lysyl-tRNA synthetase LysU, E.coli), 1quqA (Replication protein A, 32kDa subunit, Human), 1quqB (Replication protein A, 14kDa subunit, Human), 1jmcA (Replication protein A, 70kDa subunit (RPA70) fragment, Human), 1otc (Telomere-end-binding protein, O. nova), 3ullA (Mitochondrial ssDNA-binding protein, Human), 1prtF (Pertussis toxin S5 subunit, B. pertussis), 1bcpD (Pertussis toxin S5 subunit (ATP bound), B. pertussis), 3chbD (Cholera Toxin, V. cholerae), 1tiiD (Heat-labile toxin, E. coli), 2bosA (Verotoxin-1/Shiga toxin, B-pentamer, E. coli), 1br9 (TIMP-2, Human), 1an8 (Superantigen SPE-C, S. pyogenes), 3seb (Superantigen SPE, S. aureus), 1aw7A (Toxic shock syndrome toxin, S. aureus), 1jmc (Major cold-shock protein, E.coli), 1bkb (Initiation translation factor 5a, P. aerophylum), 1sro (S1 RNA-binding domain of PNPase, E.coli), 1d7qA (Initiation translation factor 1, elF1a, Human), 1ah9 (Initiation translation factor 1, IF1, E.coli), 1b9mA (Mo-dependent transcriptional regulator ModE, E.coli), 1ckmA (RNA guanylyltransferase, Chlorella virus, PBCV-1), 1a0i (ATP-dependent DNA ligase, Bacteriophage T7), 1snc (Staphylococcal nuclease, S. aureus),1 hjp (DNA helicase RuvA subunit, N-terminal domain, E.coli), 1pfsA (Gene V protein, Pseudomonas bacteriophage pf3), 1gvp (Gene V protein, Filamentous bacteriophage (f1, M13)), 1gpc (Gene 32 protein (gp32) core, Bacteriophage T4), 1wgjA (Inorganic pyrophosphatase, S. cerevisiae), and 2prd (Inorganic pyrophosphatase, T. thermophilus).

Non-exhaustive examples of proteins with SH3 domaines are Signal transducing adaptor proteins, CDC24, Cdc25, PI3 kinase, Phospholipase, Ras GTPase-activating protein, Vav proto-oncogene, GRB2, p54 S6 kinase 2 (S6K2), SH3D21, C10orf76 (potentially), STAC3, Some myosins, SHANK1,2,3, ARHGAP12, C8orf46, TANGO1, Integrase, Focal Adhesion Kinase (FAK, PTK2), Proline-rich tyrosine kinase (Pyk2, CADTK, PTK2beta), or TRIP10 (cip4).

### Multimeric proteins

Among the preferred multimeric protein, one can cite the proteins of the TNF superfamily. The tumor necrosis factor (TNF) superfamily is a protein superfamily of type II transmembrane proteins containing TNF homology domain and forming trimers.

Members of this superfamily can be released from the cell membrane by extracellular proteolytic cleavage and function as a cytokine (such as TNF-alpha, which is involved in inflammation).

These proteins are expressed predominantly by immune cells and regulate diverse cell functions, including regulation of immune response and inflammation, but also proliferation, differentation, apoptosis and embryogenesis.

The superfamily contains 19 members (Lymphotoxin alpha, Tumor necrosis factor, Lymphotoxin beta, OX40 ligand, CD40 ligand, Fas ligand, CD27 ligand, CD30 ligand, CD137 ligand, TNF-related apoptosis-inducing ligand, Receptor activator of nuclear factor kappa-B ligand (RANKL), TNF-related weak inducer of apoptosis, A proliferation-inducing ligand, B-cell activating factor, LIGHT, Vascular endothelial growth inhibitor, TNF superfamily member 18, and Ectodysplasin A).

These proteins bind to receptors that are members of TNF receptor superfamily, which comprises 29 members.

Among the proteins of the TNF superfamily, specific mention is made of
- TNF-alpha, which binds to Tumor necrosis factor receptor 1 (1A), Tumor necrosis factor receptor 2 (1B) or Lymphotoxin beta receptor
- RANKL, which binds to RANK, Osteoprotegerin or TWEAK receptor
- TRAIL (TNFSF10), which interacts with TNFRSF10B.

Tumor necrosis factor (TNF, tumor necrosis factor alpha, TNFα, cachexin, or cachectin) is a cell signaling protein (cytokine) involved in systemic inflammation and is one of the cytokines that make up the acute phase reaction. It is produced chiefly by activated macrophages, although it can be produced by many other cell types such as CD4+ lymphocytes, NK cells, neutrophils, mast cells, eosinophils, and neurons. Dysregulation of TNF production has been implicated in a variety of human diseases including Alzheimer's disease,[6] cancer,[7] major depression,[8] psoriasis[9] and inflammatory bowel disease (IBD).

Some antibodies against TNF have been developed to treat various diseases:
- Adalimumab is used to treat rheumatoid arthritis, psoriatic arthritis, ankylosing spondylitis, Crohn's disease, ulcerative colitis, chronic psoriasis, hidradenitis suppurativa, and juvenile idiopathic arthritis
- Certolizumab pegol (CDP870) is used for the treatment of Crohn's disease, rheumatoid arthritis, psoriatic arthritis and ankylosing spondylitis
- Infliximab is used for the treatment of Crohn's disease, ulcerative colitis, psoriasis, psoriatic arthritis, ankylosing spondylitis, and rheumatoid arthritis. It is also used for Behçet's disease and other conditions. It is to be noted that Infliximab is administered by intravenous infusion, and cannot be given orally because the digestive system would destroy the drug.
- golimumab is used a treatment for rheumatoid arthritis, psoriatic arthritis, ankylosing spondylitis, and ulcerative colitis, and is used by subcutaneous injection or intravenous injection
- Etanercept is used to treat rheumatoid arthritis, juvenile rheumatoid arthritis and psoriatic arthritis, plaque psoriasis and ankylosing spondylitis.

TNF acts in particular by promoting release of other cytokines including IL-1, IL-6, GM-CSF, and IL-10, inducing chemokines, increasing adhesion molecules, permitting growth of blood vessels, and allowing release of tissue destructive enzymes. It also activates T cells.

TNF may also play a role in cancer by promoting an inflammatory microenvironment around the tumor, therefore limiting the efficacy of treatments (chemotherapy, CAR-T cells...).

Consequently, a variant of an OB-fold protein (in particular from the Sac7d family), having the ability to inhibit formation of a functional TNF trimer, can be used in the same applications as the above antibodies, and for treating the same diseases. Furthermore, it is advantageous to use such variants, as they can be orally or topically (applied to the skin) dispensed to the patient, an mode of administration that is not available for antibodies.

Another advantage is an enhanced long-term safety profile thanks to the capacity to selectively neutralize the activity of the soluble TNFalpha over its membrane-bound format. Blocking the membrane-bound TNF is indeed suspected of inducing (i) immune response against the blocking agent (Deora et al, MABS. 2017; 9(4):680-695) and (ii) undesired side effects such as Multiple Sclerosis.

Receptor activator of nuclear factor kappa-B ligand (RANKL) is an apoptosis regulator gene, a binding partner of osteoprotegerin (OPG), a ligand for the receptor RANK and controls cell proliferation by modifying protein levels of Id4, Id2 and cyclin D1. It is supposed to be involved in formation of bone metastases of breast or prostate cancers. Overproduction of RANKL is also implicated in a variety of degenerative bone diseases, such as rheumatoid arthritis and psoriatic arthritis. Denosumab is an antibody to RANKL, used to treat postmenopausal patients suffering with osteoporosis (PMO), and which can also be used to reduce cancer treatment-induced bone loss in breast and prostate cancers. It can also be used in multiple myeloma to prevent the development of bone complications.

Consequently, a variant of an OB-fold protein (in particular from the Sac7d family), having the ability to inhibit formation of a functional RANKL trimer, can be used in the same applications, and for treating or preventing the same diseases or complications.

TNF-related apoptosis-inducing ligand (TRAIL), is a protein functioning as a ligand that induces the process of cell death called apoptosis. TRAIL and its receptors have been used as the targets of several anti-cancer therapeutics. TRAIL has also been implicated as a pathogenic or protective factor in various pulmonary diseases, particularly pulmonary arterial hypertension. It has been shown recently that neutralization of TNFSF10 ameliorates functional outcome in a murine model of Alzheimer's disease (Cantarella et al, Brain. 2015 Jan; 138(1): 203-216). Consequently, a variant of an OB-fold protein (in particular from the Sac7d family), having the ability to inhibit formation of a functional TRAIL trimer, can be used for the treatment of Alzheimer disease, for treating the disease or preventing further progression of it.

### Method for screening for a Sac7d variant

The invention also provides a method for obtaining a protein that binds to a monomer of a multimeric protein comprising the steps of:
a. Providing a combinatorial library of variants of a protein of the Sac7 family, in which 5 to 22 residues of the binding interface of said OB-fold protein with its native ligand have been randomized, wherein said OB-fold protein is Sac7d or Sac7e from Sulfolobus acidocaldarius, or Sso7d from Sulfolobus solfataricus, or DBP 7 from Sulfolobus tokodaii, or Ssh7b from Sulfolobus shibatae, or Ssh7a from Sulfolobus shibatae, or p7ss from Sulfolobus solfataricus, wherein said residues that are randomized in said variants are selected amongst the residues corresponding to V2, K3, K5, K7, Y8, K9, G10, E14, T17, K21, K22, W24, V26, G27, K28, M29, S31, T33, D35, D36, N37, G38, K39, T40, G41, R42, A44, S46, E47, K48, D49, A50 and P51 of Sac7d
b. Expressing said variants (in particular by ribosome, yeast display)
c. Presenting said variants to said multimeric protein that has been conjugated to a solid support so that multimerization has been abolished, and wherein the face which is involved in multimerization of the multimeric protein is being presented and available for binding
d. Selecting variants that bind to said face
e. Obtaining a sub-library of said selected variants
f. Optionally repeating steps (b) to (e) from one to three time
g. Selecting a variant of said sub-library wherein said variant comprises 5 to 25 residues mutated in the binding interface with the native ligand of said starting protein of the Sac7d protein, and binds to said face of said multimeric protein which is involved in multimerization and inhibits multimerization of said multimeric protein

This method thus makes it possible to identify a variant that binds to the subunit and inhibits or slows the formation of the biologically functional multimer, from the library of variants.

The variant inhibits multimerization of a multimeric (whether homo or hetero multimeric) protein. In fact, it is reminded that protein-protein binding is a dynamic process and that two polypeptides shall be bound at one time and separate at another time. The main question for obtaining stable multimers is the affinity of two proteins (one for the other) which will control in particular the duration of binding of the proteins. In the present application, it is said that the variant inhibits the formation of the multimer when the equilibrium between the free monomer and the monomer bound in the multimeric protein is shifted. This is performed by competition, for the unbound monomeric form, between the variant and the other monomers. If the free unbound monomer binds the variant, there is less multimer that is formed and the equilibrium is thus shifted. The action of the variants herein disclosed is thus to be understood as being a competitor partner for an unbound free monomeric part of a multimeric protein, that will bind to such monomer at an epitope such that binding to the other natural partners will be prevented, thereby limiting the formation of the functional multimeric protein.

Step a) consists in providing a library of variants of an OB-fold or an SH3 protein, in particular of variants of protein of the Sac7d family. These variants in the library are randomly mutated at a given number of amino acids that are positioned in the binding site. The prior art (in particular WO2007139397 and WO 2008/068637) explains in details how the binding site of these proteins can be determined. In particular, one can superimpose the OB-fold or SH3 scaffold on the Sac7d scaffold and use the amino acids corresponding to the ones indicated above.

Step b) consists in the expression of the variants. Such expression is performed by any method in the art, such as bacterial, yeast or ribosome display. Ribosome display is particularly adapted. The two patent application documents provide all necessary guidance to perform this step.

In step c), the expressed variants are presented to the subunit of the multimeric protein, that are conjugated to a solid support. Such conjugation has been performed in condition such that the multimerization of the protein has been abolished (i.e. the protein is not anymore in the multimeric form), so that the face of the subunit that is involved in the multimerization of the multimeric protein (protein-protein binding wituin the multimer) is presented and thus is available for binding by the variants.

According to steps d) and e), the variants that bind to the proteins presented in step c) are selected, thereby providing a a sub-library containing said selected variants. Such selection steps are known in the art and classical in the process of identifying binders in a library.

Steps b) to e) can be repeated, generally one to three times, so as to improve convergence of the sub-library (i.e. limitation of the number of variants in sequential sub-libraries), in particular by increasing the stringency of binding (in order, notably to obtain binders with improved affinities). This would eventually improve the quality of the binders, and make it possible to eventually select very specific and affine binders (step g).

In order to achieve the goal of obtaining variants that bind to the face involved in the multimerization of the protein, step c, namely being sure that such face is presented to the variants in the library, is important.

This may be achieved by conjugating the multimeric protein to a solid ligands according to the following steps of
a. Grafting/conjugating a ligand to the multimeric protein
b. Immobilizing the grafted/conjugated protein on a solid surface via the ligand
c. Washing the surface in order to eliminate unbound multimeric protein

Such ligand grafted in a. may be biotin which can easily be used for conjugation of proteins. The fact that the ligand is conjugated to the multimeric form of the protein makes it possible to make sure that it will not bind the face of the subunits that are involved in the multimerization and stabilization of the multimer.

Immobilization of the grafted/conjugated protein on a solid surface (in particular on beads) is performed *via* the ligand. The liaison biotin/avidin (in particular using streptavidin or neutravidin on the solid surface) is particularly adapted for such step b.

Eventually, the solid surface is washed so as to eliminate both unbound multimeric protein and to "demultimerize" the protein, i.e. to eliminate the subunits that are involved in the multimer but have not be bound to the surface. This, will only remain on the surface the "lonely" subunits that are bound *via* the ligand. Such subunits will thus "present" the face involved in multimerization as the target for the variants of the library.

It is to be noted that the face involved in multimerization of a multimeric protein can be determined by crystallization of the multimer. Alternatively, or in complement, directed mutagenesis, followed by cross-linkage experiments (such as SDS-PAGE or size exclusion) makes it possible to determine the amino acids involved in protein-protein interaction and formation of the multimer.

When using the method above, the multimer are first bound to the solid surface. Making different washes eventually leads to the release of the subunit that are involved in the multimer and not bound to the solid surface, thereby presenting the face involved in the protein-protein interaction in the multimer.

In order to check that multimerization is inhibited, one can use methods known in the art: after co-incubating the isolated variant, and monomers, one can check that inhibition has been inhibited by size exclusion or SDS-PAGE. These methods are known in the art.

It is further believed that it is possible to obtain variants against any monomer involved in a multimeric protein. Indeed, the face involved in the protein-protein interaction in the multimer has evolved to present a certain tropism for protein-protein interaction and thus would be prone to an interaction with the variant of the OB-fold domain. It is thus believed that there is a.competitive advantage for this face to bind to the variants that are presented in the display.

### Production of the identified variants

The sequence of the identified variant can be cloned in any appropriate vector by any molecular genetic methods known in the art.

These recombinant DNA constructs comprising a nucleotide sequence, coding for a polypeptide comprising a variant as described above, are used in connection with a vector, such as a plasmid, phagemid, phage or viral vector.

These recombinant acid molecules can be produced by techniques described in Sambrook et al., 1989 (Sambrook J, Fritschi EF and Maniatis T (1989) Molecular cloning: a laboratorymanual, Cold Spring Harbor Laboratory Press, New York). Alternatively, the DNA sequences may be chemically synthesized using, for example, synthesizers.

Recombinant constructs of the invention comprise the expression vectors that are capable of expressing the RNA and thus lead to production of proteins from the above genetic sequences. The vector may thus further comprise regulatory sequences, including a suitable promoter operably linked to the open reading frame (ORF) of the genetic sequences herein disclosed. The vector may further comprise a selectable marker sequence such as an antibiotic resistance gene. Specific initiation and bacterial secretory signals also may be required for efficient translation of the coding sequences when bacteria as used as the expression host.

### Production of the molecules

Cells are transfected or transformed with vectors containing the sequences coding for the polypeptides comprising the variant as disclosed above.

The cells are the cultured in such conditions as to have the protein expressed and favorably secreted. The conditions of culture of the cells are the conditions generally used for recombinant antibody production and are known in the art. Such conditions that are known in the art can also be optimized by the person skilled in the art if needed. Kunert and Reinhart (Appl Microbiol Biotechnol. 2016; 100: 3451-3461) review such methods and provide ample references thereto.

One can use bacterial, phage (Shukra et al, Eur J Microbiol Immunol (Bp). 2014; 4(2): 91-98) or eukaryotic systems of production.

One shall prefer to use eukaryotic cells in order to obtain proper post-translational modifications such as glycosylation.

In particular, one can use CHO (Chinese Hamster Ovary) cells, PER.C6 cells (human cell line, Pau et al, Vaccine. 2001 21;19(17-19):2716-21), HEK 293b cells (Human embryonic kidney cells 293), NS0 cells (cell line derived from the non-secreting murine myeloma) or EB66 cells (a duck cell line Valneva, Lyons, France).

Also provided by the present disclosure are host cells containing at least one of the DNAs constructs coding for a polypeptide comprising the variant as disclosed herein. The host cell can be any cell for which expression vectors are available. As indicated above, it may be a higher eukaryotic host cell, such as a mammalian cell, a lower eukaryotic host cell, such as a yeast cell, or a prokaryotic cell, such as a bacterial cell.

Introduction of the recombinant construct into the host cell is performed by any method known in the art (such as calcium phosphate transfection, lipofection, DEAE, dextran mediated transfection, electroporation or phage infection). The vectors can be inserted within the genome of the host cell, or be maintained as an extragenomic vector (such as a Bacterial Artificial Chromosome or a Yeast Artificial Chromosome). When introduced within the cell genome, such introduction may be random or targeted using methods known in the art (homologous recombination or the like).

### Bacterial hosts and expression

Useful expression vectors for bacterial use are constructed by inserting the recombinant DNA sequence together with suitable translation initiation and termination signals in operable reading phase with a functional promoter. The vector will comprise one or more phenotypic selectable markers and an origin of replication to ensure maintenance of the vector and, if desirable, to provide amplification within the host.

Suitable prokaryotic hosts for transformation include *E. coli, Bacillus subtilis, Salmonella typhimurium* and various species within the genera *Pseudomonas, Streptomyces,* and *Staphylococcus.*

### Eukaryotic hosts and expression

Examples of the eukaryotic host cells include vertebrate cells, insect cells, and yeast cells. In particular, one can use the cells mentioned above.

The transformed or transfected cells are cultured according to methods known in the art and the polypeptide are recovered from intracellular or extracellular fractions (depending on whether it is secreted or not).

### Isolation of the molecules

The recombinant multi-specific protein produced can be separated and purified by any of various known separation methods utilizing the physical or chemical property of the protein, from the intracellular or extracellular fraction.

In particular, one can use methods such as precipitation, ultrafiltration, various types of liquid chromatography such as molecular sieve chromatography (gel filtration), adsorption chromatography, ion exchange chromatography, and affinity chromatography, dialysis, and a combination thereof.

In general, any method known and used to purify recombinant polypeptides is adapted for the purification of the molecules herein disclosed.

If a tag has been introduced within the recombinant sequence (such as a poly-Histidine tag), one can purify the molecules using this tag. However, it is preferred to use affinity to purify the molecules.

One can, in particular, use the fact that the molecule herein produced binds to a specific target and use any affinity method (affinity column, FACS, beads) to isolate such molecules.

One particular advantage of the molecules herein disclosed is that they don't need to be glycosylated to be active and can thus be produced in any type of cells, and not necessarily eukaryotic cells. They are particularly well produced in bacterial cells.

### Modification of the variants

The variants isolated, in particular by the method disclosed above, and having the ability to shift the formation of the multimeric form of the target can be modified by any method known in the art.

### Preparing a polypeptide comprising the variant

It is possible to prepare a DNA sequence that would contain two coding sequences, one for the variant herein disclosed and another for a protein or peptide of interest. The resulting expressed protein would thus be a polypeptide that would contain both proteins. The vector may be built in such a way that it would contain a sequence coding for a linker that would be located between the two proteins in the expressed polypeptide.

Consequently, the invention also encompasses a polypeptide comprising the variant of a protein of the OB-fold protein (preferably of the Sac7d family) binding to the subunit of a multimeric protein which is linked (preferably through amine bound as disclosed above) to another protein or polypeptide.

In a specific embodiment, the other protein or polypeptide comprises another variant of a protein of the Sac7d family.

In another embodiment, the other protein or polypeptide is an antibody. In this embodiment, the variant of the OB-fold domain is fused to at least one of the heavy or light chain of an immunoglobulin monomer, preferably at the N- or C-terminus of the light or heavy chain. In another embodiment, the variant may be fused to both heavy or light chains.

In order to obtain such compounds, one can use a genetic construct comprising a DNA sequence selected from the group consisting of
a. the sequence coding for the heavy chain of an antibody fused, at its 3'end with the sequence coding for the variant of an OB-fold protein (potentially with an sequence coding for a linker)
b. the sequence coding for the heavy chain of an antibody fused, at its 5' end with the sequence coding for the variant of an OB-fold protein (potentially with an sequence coding for a linker)
c. the sequence coding for the light chain of an antibody fused, at its 3' end with the sequence coding for the variant of an OB-fold protein (potentially with an sequence coding for a linker)
d. the sequence coding for the light chain of an antibody fused, at its 5' end with the sequence coding for the variant of an OB-fold protein (potentially with an sequence coding for a linker).

This fusion can be made at the N-terminus and/or the C-terminus of the antibody chain (heavy and/or light chain). It is to be noted that, especially when using a small OB-fold domain (about 70 amino acids) such as a protein from the Sac7d family, it is possible to obtain a molecule that will have the structure of the antibody (two light chains paired to two heavy chains, and such dimers paired together), with the antibody regions, and further binding regions consisting of the modified OB-fold domain.

In a specific embodiment, the antibody part of the protein herein disclosed is a IgG molecule.

In another embodiment, the antibody part of the protein herein disclosed is a IgA molecule.

In another embodiment, the antibody part of the protein herein disclosed is a IgM molecule.

In another embodiment, the antibody part of the protein herein disclosed is a IgD molecule.

In another embodiment, the antibody part of the protein herein disclosed is a IgE molecule.

The antibody may be a human antibody, a rodent antibody (such as a mouse antibody or a rat antibody), a cat antibody, a dog antibody, a chicken antibody, a goat antibody, a camelid antibody (such as a camel antibody, a llama antibody, an alpaca antibody or a nanobody), a shark antibody, or an antibody from any other species. It may be a chimeric or humanized antibody. As reminded in Wikipedia, humanized antibodies are antibodies from non-human species whose protein sequences have been modified to increase their similarity to antibody variants produced naturally in humans. A chimeric antibody contains sequences from different species.

It is preferred when the antibody that is part of the molecule herein disclosed is an antibody that comprises two identical heavy chains (of about 400 to 500 amino acids, generally around 450 amino acids) and two identical light chains. Consequently, the antibody comprises the same Fab variable regions. This antibody is therefore a monospecific antibody where both parts (combination of light and heavy chains) of the antibody bind to the same epitope of an antigen.

However, the antibody may present different heavy and/or light chains. In particular, in some embodiments, the antibody is a bi-specific antibody. The term "antibody" thus encompasses both "classical antibodies" as disclosed above having identical heavy and light chains, but also engineered antibodies that have more than one specificity.

In a specific embodiment, the antibody presents one heavy and light chain from one antibody and another heavy and light chain from another antibody.

The antibody may bind to a target selected in the group consisting of:

Cell surface receptors: Insulin receptor, Low density lipoprotein receptor-related protein 1, Transferrin receptor, Epidermal growth factor receptor, Epidermal growth factor receptor variant III, Vascular endothelial growth factor receptor 1, Vascular endothelial growth factor receptor 2, Her2, Her3, Her4, PMSA, IGF-1R, GITR, RAGE, CD28.

Cell surface proteins: Mesothelin, EpCam, CD19, CD20, CD38, CD3, TIM-3, CEA, cMet, ICAM1, ICAM3, MadCam, a4b7, CD7, CD4, CD138.

Angiogenesis factors and Growth factors: VEGF, Angiopoietin 2, HGF, PDGF, EGF, GM-CSF, HB-EGF, TGF

Immune checkpoint inhibitors or activators: PD-1, PD-L1, CTLA4, CD28, B7-1, B7-2, ICOS, ICOSL, B7-H3, B7-H4, LAG3, KIR, 4-1BB, OX40, CD27, CD40L, TIM3, A2aR

Circulating proteins: TNFa, IL23, IL12, IL33, IL4, IL13, IL5, IL6, IL4, IFNg, IL17, RANKL, Bace1, alpha Synuclein, Tau, amyloid.

It is particularly envisaged when the antibody targets IL17 or a cell surface receptor in particular involved in cancer.

### Hence are particularly foreseen

- A polypeptide comprising a variant as disclosed above, binding to TNF-alpha and an antibody binding to IL17 (such as reference antibody secukinumab)
- A polypeptide comprising a variant as disclosed above, binding to TNF-alpha and an antibody binding to Her2/neu (reference antibodies binding to Her2/neu are trastuzumab and pertuzumab)
- A polypeptide comprising a variant as disclosed above, binding to RANKL and an antibody binding to TNF-alpha (reference antibodies binding to TNF-alpha are infliximab, adalimumab, certolizumab pegol, ozoralizumab and golimumab).

Alternatively, the polypeptide may contain a variant as disclosed above, and a biologically active molecule, such as an erythropoietin, an interferon, or etanercept.

In another embodiment, the variant of the OB-fold domain (in particular the variant of a protein of the Sac7d family) is conjugated to an organic molecule. This may be done by any method known in the art. In particular, one can chemically link the molecule to the protein. As a molecule, one can cite antiproliferative (cytotoxic and cytostatic agents) include cytotoxic compounds (e.g., broad spectrum), angiogenesis inhibitors, cell cycle progression inhibitors, PBK/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, RNA polymerase inhibitors and proteasome inhibitors. One can also use anti-inflammatory molecules.

One can cite in particular DNA-binding or alkylating drugs such as anthracyclines (doxorubicin, epirubicin, idarubicin, daunorubicin) and its analogs, alkylating agents, such as calicheamicins, dactinomycines, mitromycines, pyrrolobenzodiazepines, and the like. One can also cite cell cycle progression inhibitors such as CDK inhibitors, Rho-kinase inhibitors, checkpoint kinase inhibitors, aurora kinase inhibitors, PLK inhibitors, and KSP inhibitors. One can also cite thalidomide and its derivatives lenalidomide and pomalidomide. In order for treating inflammation disorders, one can also use cyclooxygenase-2 inhibitors, 5-lipoxygenase inhibitors, quercetin and/or resveratrol as molecules conjugated to the polypeptide comprising the variant.

### Use of the variants

The variants can be used in particular in therapeutic methods.

In particular, a variant targeting the TNFalpha would target the monomer or dimer form of the protein and can be used, in particular, for the treatment of inflammatory diseases (such as Irritable Bowel Disease (IBD), rheumatoid arthritis (RA), psoriasis and the like) or for the treatment of the diseases mentioned above.

A variant targeting RANKL would target the monomer or dimeric form and can be used in particular for the treatment of inflammatory diseases (such as the ones listed above), or of bone diseases (such as osteoporosis), or for the treatment of the diseases mentioned above.

A variant targeting the TRAIL protein would target the monomeric or dimeric form and be used for the treatment of Alzheimer's disease, or for the treatment of the diseases mentioned above.

The invention thus relates to methods of treatment of the diseases as mentioned above, comprising the administration of a therapeutic amount of a variant of an OB-fold (in particular a variant of a protein of the Sac7d family) to a patient in need thereof.

The term "therapeutic amount" or "effective amount", as used herein, is the amount sufficient to effect beneficial or desired results, such as clinical results, and an "effective amount" depends upon the context in which it is being applied. An effective amount is an amount that provides therapeutic improvement while minimizing side or adverse effect.

One can administer the variant by any method of the art.

In particular, one can inject the variant. In another embodiment, one can apply the variant topically (either on the skin or on the eye of the patient) as disclosed in WO 2014/173899. In another embodiment, one can administer the variant orally, as disclosed in WO 2016/062874.

The variants or polypeptides containing the variants can also be used in diagnostic methods.

The invention thus also relates to a method for detecting the presence of, or for quantifying, a subunit of a multimeric protein in a sample, comprising the step of
a. Exposing the sample to a variant as disclosed, that binds to the subunit but not the fully formed multimeric protein, in such conditions that such binding is possible
b. Recovering the variant and/or detecting, or measuring the amount of, quantifying, ELISA, fluorescence, columns] the subunit.

The recovery of b) can be perfomed by various washes or methods common in the art. The detection or quantification can be performed by any method such as ELISA or other methods in the art.

The invention also encompasses a method for purifying a subunit of a multimeric protein in a sample, comprising the step of
a. Exposing the sample to a variant as disclosed, that binds to the subunit but not the fully formed multimeric protein, in such conditions that binding is possible
b. Recovering the variant bound to the subunit
c. Eluting the subunit from the variant

Step c can be performed in particular by changing buffer composition (increasing/decreasing salts concentration, detergents, chaotropic agents) or pH (increasing/decreasing).

In these methods, it may be interesting to use a polypeptide comprising the variant fused to another protein that can thus be used to recover the variant bound to the subunit (for instance on columns affine for this other protein).

### DESCRIPTION OF THE FIGURES

Figure 1: alignment of proteins of the Sac7d family.
Figure 2: representation of the binding curves of N11 to TNF-alpha (TNFa), in presence of TNF Receptor I (TNFRI), TNF Receptor II (TNFRII) Adalimumab (ADA) and Infliximab (IFX).
Figure 3: OD450nm representing binding of N11 in presence of human TNFa (hTNFa), murine TNFa (mTNFa) or no target.
Figure 4: OD450nm representing binding of variants of N11 in presence of human TNFa
Figure 5: schematic representation of TNFa mutants. The E146A and Y115A are part of the ADA epitope.
Figure 6: OD450nm representing binding of N11 in presence of mutants of human TNFa (Y151A, Y115A, E146A and R32A).
Figure 7: Analysis of the functionality of the Y151A mutant of the TNFalpha as compared to the wild type of TNFa (TNF WT).
Figure 8: SDS-PAGE showing the presence of a complex of N11 with a dimer or a monomer of TNFa (arrows).
Figure 9: schematic representation of the transition between monomeric, dimeric and trimeric forms of TNFa, and of the type of mutants generated. The S95C/G148C mutant is locked in its trimeric form, whereas the Y119A and Y119R mutants are impaired for their ability to self-assemble.
Figure 10: illustration of the number of species of TNFa (monomer, dimer, trimer) for wild-type (WT) and mutant TNFa.
Figure 11: biological functionality of WT and mutants TNFa in the L929 assay.
Figure 12: OD450nm representing binding of N11 on the Y119 mutants, wild-type (WT) or no target (A) or of the other TNFa mutants, wild-type (WT) or no target (B)
Figure 13: blood measurement of IL6 in mice, 6h after administration of TNFa co-incubated with various molecules: N11, N9 variant of Sac7d binding TNFa, N9mut42A (the N9 variant with a mutation to abolish binding to TNF) and a irrelevant variant of Sac7d (Irr NF).

### EXAMPLES

### Example 1. Production of a NF against TNF or RANK Ligand

The screening was performed using a library comprising random mutations in 17 positions of the sequence of Sac7d (SEQ ID NO: 1).

The mutated positions were K7, Y8, K9, E11, K21, K22, W24, V26, M29, S31, T33, D35, T40, G41, R42, A44, and S46.

The targets were TNF-alpha and RANKL.

The targets, in the trimeric soluble form, were biotinylated and attached via biotin/avidin liaison on solid beads. They were then thoroughly washed in order to dissociate the trimers.

Screening essentially as disclosed in WO 2008/068637 was performed by ribosome display expression of the variants of the library and exposition to the bound targets.

For the selection and the identification of the clones, biotinylated hTNFa (R&D systems) and mTNFa (R&D systems) were used. The biotinylation was performed by incubation of a 50 µM solution of the target protein with a 5-fold molar excess of sulfosuccinimidyl-6-(biotinamido) hexanoate (Sulfo-NHS-LC-LC-Biotin, Pierce) in PBS (Sigma-Aldrich) on ice for 1 h. The biotinylated proteins were buffer-exchanged using protein desalting spin columns from Pierce equilibrated in 20 mM Tris-HCI, 150 mM NaCl pH 7.4 (TBS). The degree of biotinylation was determined, using the HABA assay (Sigma), as being about 0.5 to 5 molecules of biotin per protein molecule. The generation of the library corresponding to the random mutagenesis of positions as indicated above in Sac7d protein has been previously described (in particular in Mouratou et al, Methods Mol Biol. 2012;805:315-31; Mouratou et al, Proc Natl Acad Sci U S A. 2007 Nov 13; 104(46): 17983-8; WO 2008/068637).

The PCR-amplified library was transcribed and the selection was done at 4°C essentially as previously described (Mouratou et al, 2007 and 2012, *op.cit.,* Binz et al, Nat Biotechnol 2004, 22:575-582). For the first two rounds of selection, streptavidin (66 nM, 100 µl/well; Sigma-Aldrich) or neutravidin (66 nM, 100 µl/well; Thermo Scientific) in TBS150 (50 mM Tris HCl, pH 7.4, 150 mM NaCl) was alternately immobilized on a Maxisorp plate (Nunc) by overnight incubation at 4 °C. The wells were then blocked with 300 µl 0.5% BSA (Sigma-Aldrich) in TBS150 for 1 h at 4 °C. Biotinylated hTNFa (100 µl, 0.1 µM) in TBS150 with 0.5% BSA was allowed to bind for 1 h at 4 °C . Before the ribosome-display round, the wells were extensively washed with washing buffer WBT (50 mM Tris acetic acid, pH 7.5, 150 mM NaCl, 50 mM Mg(CH3COO-)2, 0.05% Tween 20). Round 3 and 4 were performed similarly except that the target was presented alternately on streptavidin or neutravidin coated magnetic beads.

A ribosome-display round consisted of a 1 h pre-panning steps on streptavidin (or neutravidin) coated material and a 1 h binding step on the target protein. After washing, RNA purification and reverse transcription, the DNA pool was amplified by RT-PCR

Multiple variants were obtained for each target and further characterized to show that they bind to the target in other assays.

One assay to verify the binding to the target is performed using a variant produced with a His-Tag.

Biotinylated antigens are immobilized on streptavidin-coated plates as described above, except that the blocking step is performed for 1h at room temperature. For the identification of the positive clones, 100 µl of E. coli crude extracts are applied to wells with or without immobilized antigen for 1 h at RT. The binding is visualized using anti-RGSHis antibody HRP conjugate (Qiagen) which detects RGSHis6 tag from the variant and a solution at 1 mg/ml of o-phenylenediamine substrate (OPD) in revelation buffer (0.05 M citric acid, 0.05% hydrogen peroxide). Absorbance at 450 nm is measured using an ELISA plate reader. Quantitative ELISAs is done in the same manner, except purified protein is used.

Characterization of a specific variant binding to TNF-alpha, called N11, is disclosed in more details in the following examples.

### Example 2. Further characterization of a specific variant (N11)

### Example 2.1 Competition with TNF receptors and known binders

The ability of other binders to TNF-alpha to compete with the binding of N11 was assessed.

Briefly, the other binders (TNF Receptor I (TNFRI), TNF Receptor II (TNFRII) or two monoclonal antibodies Adalimumab (ADA) and Infliximab (IFX)) were co-incubated with N11 and the soluble TNF.

As shown in Figure 2, binding of N11 is neutralized by TNFRI, TNFRII, and Adalimumab whereas binding of N11 is not neutralized by Infliximab

### Example 2.2. Cross binding between human and mouse TNF

EC100 for N11-human TNFa (hTNFa) interaction was determined by ELISA using plate functionalized with hTNFa and a concentration range of N11.

Cross reactivity of N11 on hTNFa and mTNFa (murine TNFa) was also monitored by ELISA at a single concentration (EC100). A plate was functionalized with hTNFa and mTNFa. Wells not functionalized with TNFa have been used as negative control to highlight the specifity of the signal observed.

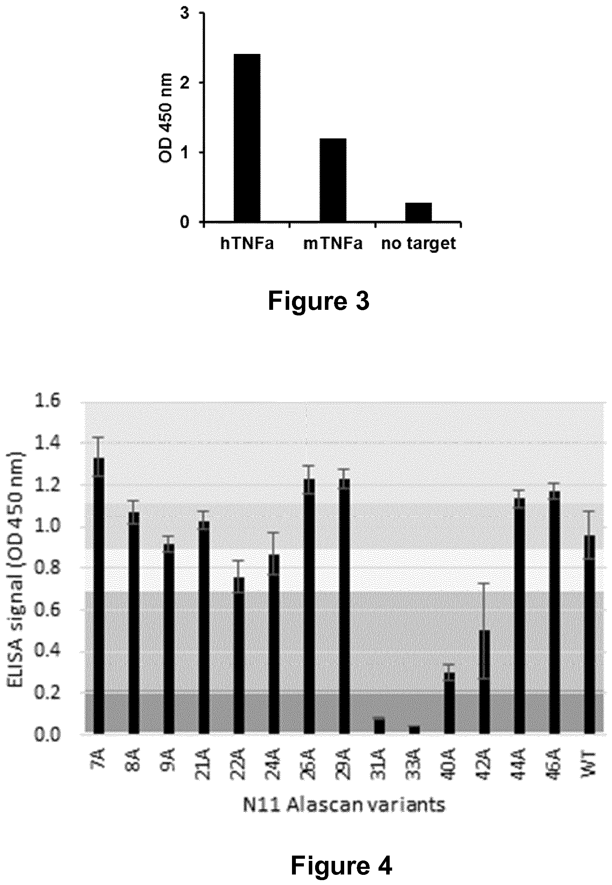

It was shown that N11 does cross-react between human and mouse TNF (Figure 3).

### Example 2.3. Scan for important residues.

In order to determine which residues are responsible for the observed binding, each residue of N11 (SEQ ID NO: 16) differing from the Sac7d (SEQ ID NO: 1) sequence was replaced by an Alanine (Alascan).

Exposition of the obtained proteins to TNFa was then performed and binding was measured by ELISA (measure of EC100 using plate functionalized with hTNFa and a concentration range of the variants of N11).

If there is a loss of binding, this indicates that the residue is very important for the binding to the subunit.

As seen in Figure 4, it was shown that residues 31, 33, 40 and 42 are very important.

Residues 22 and 24 are also important, although mutations thereof would essentially reduce the affinity.

Modification of the other mutated residues doesn't essentially impact the binding.

It thus appears that sequences SEQ ID NO: 17 (including the very important amino acids 31, 33, 40, 42 of N11) and SEQ NO: 18 (including the very important amino acids 31, 33, 40, 42 and residues 22 and 24 of N11) represent minimal sequences that maintain the binding to TNFa.

### Conclusion

The above information
- Binding human and mouse TNFa
- Competition with ADA, TNFRI, TNFRII but not IFX
- nature of the important amino acids
was used for *in silico* modeling, that showed that N11 would likely bind to the trimerisation core of TNF (two models were obtained, both binding to the core). This was further demonstrated experimentally by engineering mutants of TNFalpha by directed mutagenesis (example 3).

### Example 3. Design of TNF mutants and further characterization

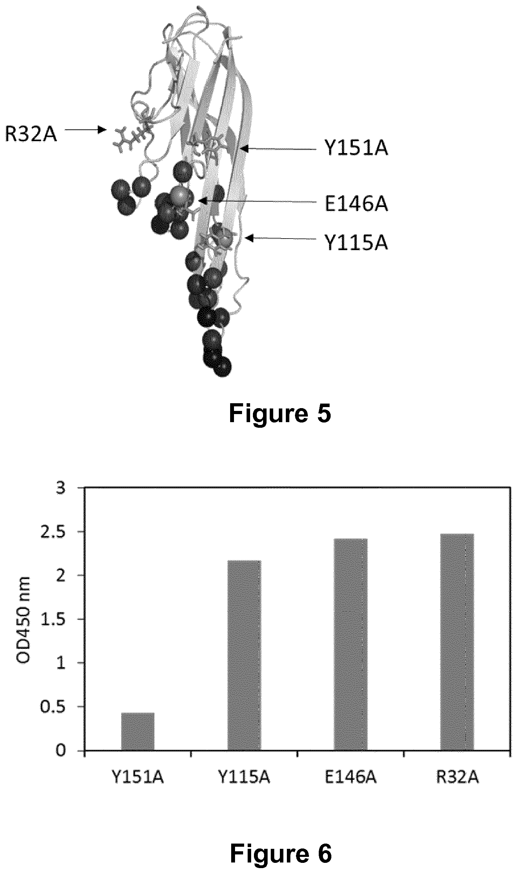

4 single mutants of TNFalpha were generated (Figure 5)
- TNFvariant R32A
   - TNFvariant E146A
   - TNFvariant Y115A
   - TNFvariant Y151A

The binding of N11 on plates coated with TNFalpha variants was checked (Figure 6). It was shown that binding was essentially abolished for mutant Y151A.

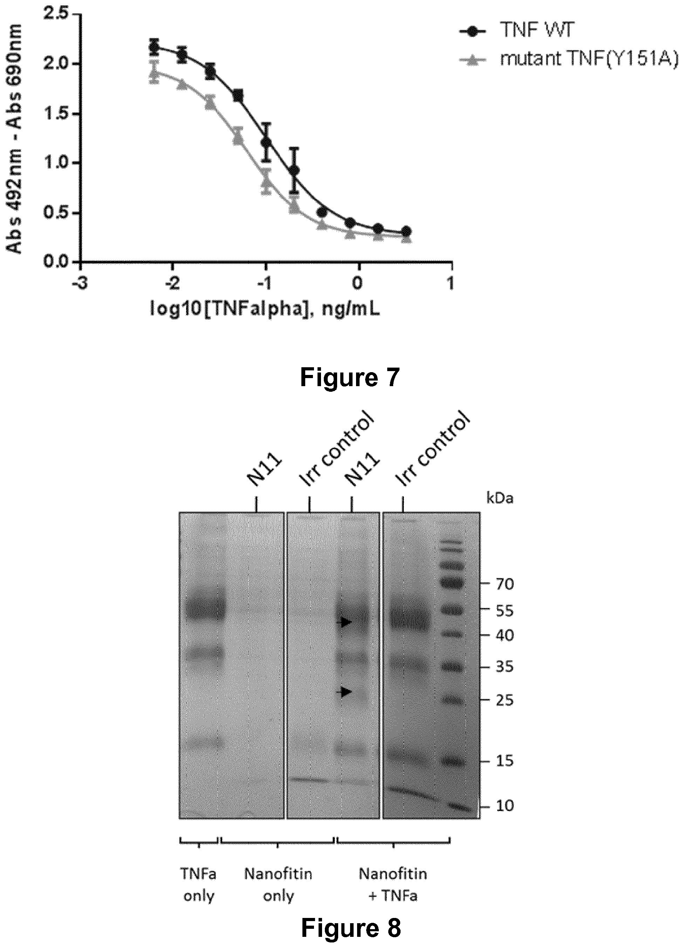

It was however shown that Y151 mutant was still functional (Figure 7).

This was performed by the L929 assay.

This indicates that the mutant Y151A is most likely well folded and that the residue is involved in the epitope recognized by N11.

### Conclusion

N11 binding is significantly impacted by Y151A mutation.

Y151A mutant was demonstrated as being fully functional.

Residue Y151 is involved in the epitope of N11
It appears that residue Y151 is burried in the core of the TNF alpha trimer
This is an additional argument pointing on a mode of interaction relying on the blockade of the TNFalpha trimerization

### Example 4. Further binding analysis - Inhibition of formation of trimer

### Cross linking experiments were performed

Figure 8 shows that bands could be attributed to a complex of N11 with a monomer and a dimer of TNFa were observed on SDS-PAGE gel after cross-linking, whereas there was no evidence of an interaction between N11 and the trimer of TNFa. It was further observed complexes of lower molecular weight when TNFa is incubated with N11 for 24h at RT, that could be attributed to a monomer of TNFa in complex with one Nanofitin and a dimer of TNFa in complex with one Nanofitin.

In a similar cross linking experiment where TNF was co-incubated with N11 overtime, it was observed that rates of TNFa trimer and dimer decreased as the time of incubation increased, whereas rates of TNFa monomer and dimer in complex with N11 increased as the time of incubation increased.

Similarly to what was observed in the cross linking experiments, species of lower molecular weight appeared after incubation of TNFa with N11.

### Example 5. Inhibition of trimer formation

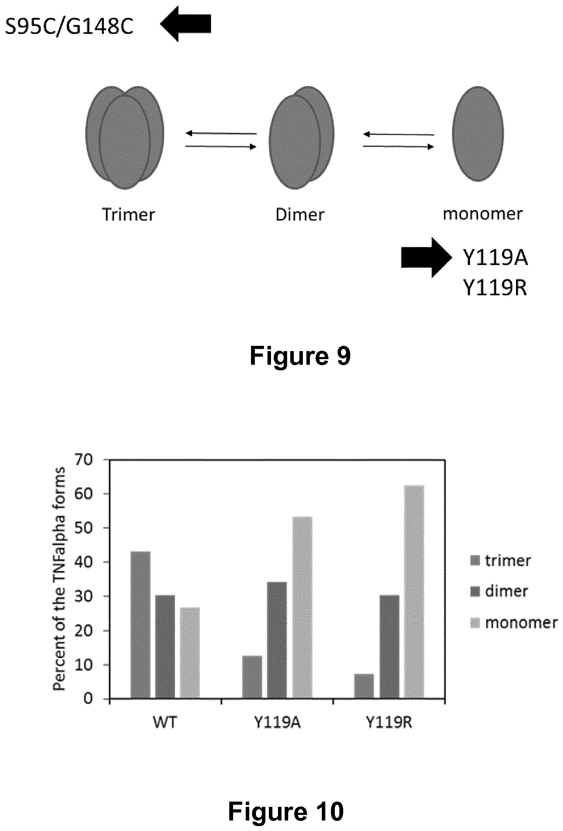

The above data strongly supports the hypothesis that N11 bind both the monomer and dimer of TNFa but not the trimer.
Mutants of TNFa were engineered to strengthen this finding (Figure 9).
a) mutants that generate a stabilized trimer of TNFa
   The stabilization of the trimer is achieved by the formation of disulfide bridges via the double mutation S95C/G148C
b) Mutants that are impaired for trimerization
   Position 119 is critical for the trimerization of TNFalpha. Mutations at the position 119 impair the trimer formation
   A low proportion of trimer observed in Y119 mutants compared to WT TNFalpha (figure 10).

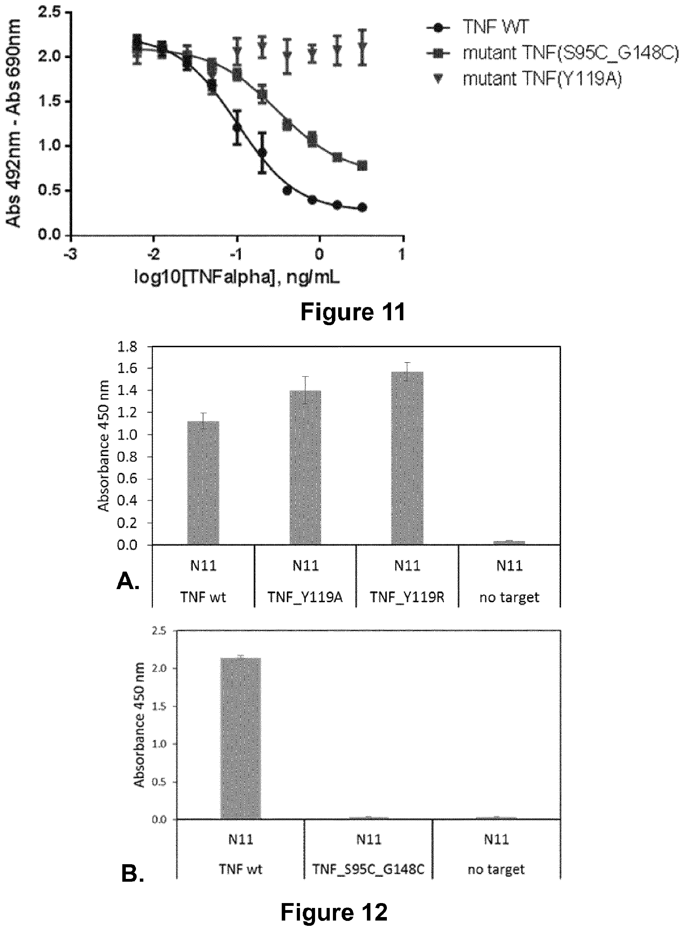

The functionality of the TNFa mutants was verified (Figure 11).
The stabilized trimer remained functional (Mutant S95C/G148C) in L929 assay
The mutants Y119 were not functional in L929 assay. This observation was expected as the mutation at this position alters the trimerization and the active form of TNFalpha is the trimer. The L929 assay being not relevant to confirm that the mutations at Y119 do not impair with a correct folding of the respective TNFalpha mutants, other tests were performed to verify the functionality of Y119 mutants. A binding assay with Infliximab showed that a similar binding of infliximab (IFX) was observed on WT and Y119 TNFalpha variants. Since the Y119 mutants are still functional for binding to IFX, this indicated that the proteins are well folded.
It was shown that N11 does not bind the trimer but remains fully capable of binding the TNFalpha in its lower oligomeric states (Figure 12). The absence of binding on the stabilized TNFa trimer correlates well with the observation made from the cross linking experiments
Binding of N11 for TNFa could also be monitored on Octet RED96 with Y119 mutants but not with WT TNFa (not shown)

### Conclusions

Cross linking experiments have shown that N11 binds both monomer and dimer of TNFalpha but not the trimer. Epitope of N11 involves a residue that is burried in the core of TNFalpha trimer. N11 cannot bind the stabilized trimer of TNFalpha. Binding of N11 is facilitated when TNFalpha is present in its lower oligomeric states

### Example 6. In vivo experiments

TNFa neutralization by N11 was demonstrated in an *in vivo* set up
A mixture of TNFa and N11, pre-incubated for 24h was administered i.v. at mice and the measure of IL6 (cytokine that is produced in response to TNFa) in the blood was performed after 6 h.
Further controls were used: N9, another (less efficient) variant isolated in Example 1, N9mut42A (the N9 variant with a mutation to abolish binding to TNF) and a irrelevant variant of Sac7d (Irr NF).

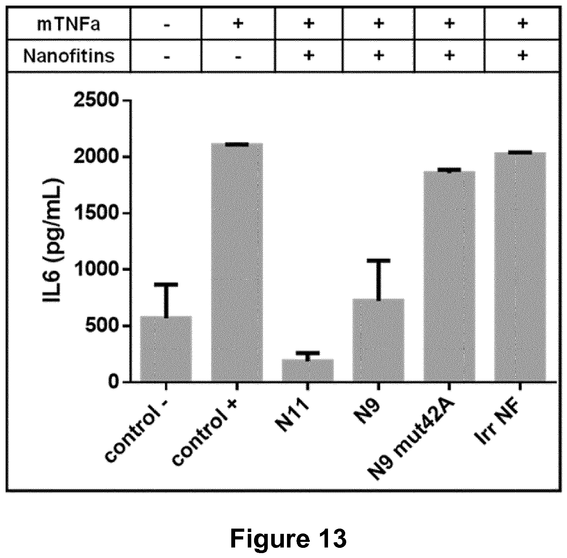

Figure 13 shows that both N11 and N9 are able to abolish production of IL6 *in vivo* thereby inhibiting the activity of TNFa *in vivo.*
Other *in vivo* experiments showed efficacy of various variants used orally in a murine model of acute colitis.

## Claims

1. A polypeptide comprising a variant of a protein of the Sac7d family, the variant comprising between 4 and 22 mutated amino acids in the binding site of the protein of the Sac7d family, that binds to a subunit of a multimeric protein and that doesn't bind to the subunit of the protein when such subunit protein is involved in the fully formed multimeric protein in its native state.

2. The polypeptide of claim 1, wherein the mutated amino acids of the Sac7d variant are selected from the group consisting of V2, K3, K5, K7, Y8, K9, G10, E11, K13, E14, T17, K21, K22, W24, V26, G27, K28, M29, S31, T33, Y34, D35, D36, N37, G38, K39, T40, G41, R42, A44, S46, E47, K48, D49, A50 and P51 of Sac7d.

3. The polypeptide of claim 1 or 2, wherein the Sac7d variant contains 4 to 17 mutated amino acids selected from the group corresponding to K7, Y8, K9, E11, K21, K22, W24, V26, M29, S31, T33, D35, T40, G41, R42, A44, and S46 of Sac7d.

4. The polypeptide of any one of claims 1 to 3, wherein the multimeric protein is part of the Tumor necrosis factor superfamily, preferably selected from the group consisting of TNF-alpha, RANKL and TRAIL.

5. The polypeptide of any one of claims 1 to 4, which comprises the sequence SEQ ID NO: 17 or SEQ ID NO: 18.

6. The polypeptide of any one of claims 1 to 5, which comprises the sequence SEQ ID NO: 16.

7. The polypeptide of any one of claims 1 to 5, which comprises the sequence SEQ ID NO: 16, in which from 1 to 8 amino acids selected from the group consisting of V7, M8, F9, K11, V21, H22, M24, Q26, L29, E35, D41, F44 and P46 have been replaced by another amino acid.

8. The polypeptide of any one of claims 1 to 7, wherein the variant of a protein of the Sac7d family binding to the subunit of a multimeric protein is linked or fused to another protein or polypeptide.

9. The polypeptide of claim 8, wherein the other protein or polypeptide comprises another variant of a protein of the Sac7d family.

10. The polypeptide of claim 8, wherein the other protein or polypeptide is an antibody, preferably binding to IL17, TNF-alpha or Her2/neu.

11. The polypeptide of any one of claims 1 to 10, which is conjugated to an organic molecule.

12. A genetic construct comprising a DNA sequence coding for the polypeptide of any one of claims 1 to 10.

13. A vector comprising the genetic construct of claim 12.

14. A host cell comprising the genetic construct of claim 12 in its genome.

15. A method for producing the polypeptide of any one of claims 1 to 10, comprising the steps consisting of
a. Culturing a cell culture wherein the cells have been transformed by a genetic construct of claim 12,
and
b. Recovering the polypeptide.

16. A method for obtaining a protein that binds to a monomer of a multimeric protein comprising the steps of:
a. Providing a combinatorial library of variants of a protein of the Sac7 family, in which 4 to 22 residues of the binding interface of said OB-fold protein with its native ligand have been randomized, wherein said OB-fold protein is Sac7d or Sac7e from Sulfolobus acidocaldarius, or Sso7d from Sulfolobus solfataricus, or DBP 7 from Sulfolobus tokodaii, or Ssh7b from Sulfolobus shibatae, or Ssh7a from Sulfolobus shibatae, or p7ss from Sulfolobus solfataricus, wherein said residues that are randomized in said variants are selected amongst the residues corresponding to V2, K3, K5, K7, Y8, K9, G10, E14, T17, K21, K22, W24, V26, G27, K28, M29, S31, T33, D35, D36, N37, G38, K39, T40, G41, R42, A44, S46, E47, K48, D49, A50 and P51 of Sac7d
b. Expressing said variants
c. Presenting said variants to said multimeric protein that has been conjugated to a solid support so that multimerization has been abolished
d. Selecting variants that bind to said face
e. Obtaining a sub-library of said selected variants
f. Optionally repeating steps (b) to (e) from one to three time
g. Selecting a variant of said sub-library wherein said variant comprises 5 to 20 residues mutated in the binding interface with the native ligand of said starting protein of the Sac7d protein, and binds to said face of said multimeric protein which is involved in multimerization and inhibits multimerization of said multimeric protein

17. The method of claim 16, wherein the conjugation of the multimeric protein to a solid ligands comprises the steps of
a. Grafting/conjugating a ligand to the multimeric protein
b. Immobilizing the grafted/conjugated protein on a solid surface via the ligand
c. Washing the surface in order to eliminate unbound multimeric protein

18. A polypeptide according to any one of claims 1 to 11 as a medicament.

19. A polypeptide according to any one of claims 1 to 11 for use thereof for the inhibition of the multimerization of a multimeric protein.

20. A method for detecting the presence of, or for quantifying, a subunit of a multimeric protein in a sample, comprising the step of
a. Exposing the sample to a variant according to any one of claims 1 to 11, that binds to the subunit, in such conditions that binding is possible
b. Recovering the variant and/or detecting, or measuring the amount of, the subunit.

21. A method for purifying a subunit of a multimeric protein in a sample, comprising the step of
a. Exposing the sample to a variant according to any one of claims 1 to 11, that binds to the subunit, in such conditions that binding is possible
b. Recovering the variant bound to the subunit
c. Eluting the subunit from the variant


SEQUENCE LISTING

<110>  AFFILOGIC

<120>  BINDERS FOR INHIBITING FORMATION OF MULTIMERIC PROTEINS

<130>  BRV 97-EP PRIO

<160>  18

<170>  PatentIn version 3.5

<210>  1
<211>  66
<212>  PRT
<213>  Sulfolobus acidocaldarius

<400>  1

Met Val Lys Val Lys Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Asp Met Leu Ala Arg Ala Glu Arg Glu
    50                  55                  60

Lys Lys
65

<210>  2
<211>  64
<212>  PRT
<213>  Sulfolobus solfataricus

<400>  2

Met Ala Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  3

<211>  65
<212>  PRT
<213>  Sulfolobus acidocaldarius

<400>  3

Met Ala Lys Val Arg Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Met Asp Met Leu Ala Arg Ala Glu Lys Lys
    50                  55                  60

Lys
65

<210>  4
<211>  64
<212>  PRT
<213>  Sulfolobus shibatae

<400>  4

Met Val Thr Val Lys Phe Lys Tyr Lys Gly Glu Glu Lys Glu Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  5
<211>  64
<212>  PRT
<213>  Sulfolobus shibatae

<400>  5

Met Ala Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  6
<211>  64
<212>  PRT
<213>  Sulfolobus tokodaii

<400>  6

Met Val Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Gln Met Leu Glu Lys Ser Gly Lys Lys
    50                  55                  60

<210>  7
<211>  64
<212>  PRT
<213>  Sulfolobus islandicus

<400>  7

Met Val Thr Val Lys Phe Lys Tyr Lys Gly Glu Glu Lys Gln Val Asp
1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  8
<211>  64
<212>  PRT
<213>  Sulfolobus islandicus

<400>  8

Met Thr Thr Val Lys Phe Lys Tyr Lys Gly Glu Glu Lys Gln Val Asp

1               5                   10                  15

Thr Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  9
<211>  64
<212>  PRT
<213>  Sulfolobus islandicus

<400>  9

Met Thr Thr Val Lys Phe Lys Tyr Lys Gly Glu Glu Lys Gln Val Asp
1               5                   10                  15

Ile Ser Lys Ile Lys Lys Val Trp Arg Val Gly Lys Met Ile Ser Phe
            20                  25                  30

Thr Tyr Asp Glu Gly Gly Gly Lys Thr Gly Arg Gly Ala Val Ser Glu
        35                  40                  45

Lys Asp Ala Pro Lys Glu Leu Leu Gln Met Leu Glu Lys Gln Lys Lys
    50                  55                  60

<210>  10
<211>  62
<212>  PRT
<213>  Metallosphaera sedula.

<400>  10

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asn Met Ile Gly Lys Lys
    50                  55                  60

<210>  11
<211>  62

<212>  PRT
<213>  Metallosphaera cuprina

<400>  11

Met Ala Thr Lys Ile Lys Phe Lys Tyr Lys Gly Gln Asp Leu Glu Val
1               5                   10                  15

Asp Ile Ser Lys Val Lys Lys Val Trp Lys Val Gly Lys Met Val Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Ser Met Ile Gly Lys Lys
    50                  55                  60

<210>  12
<211>  61
<212>  PRT
<213>  Acidianus hospitalis

<400>  12

Met Thr Thr Val Lys Phe Lys Tyr Lys Gly Glu Glu Lys Glu Val Asp
1               5                   10                  15

Ile Ser Lys Ile Lys Lys Val Trp Arg Val Gly Lys Met Ile Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Glu Lys Leu Glu Lys Lys
    50                  55                  60

<210>  13
<211>  62
<212>  PRT
<213>  Acidianus hospitalis

<400>  13

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Asp Lys Leu Glu Lys Lys
    50                  55                  60

<210>  14
<211>  60
<212>  PRT
<213>  Acidianus hospitalis

<400>  14

Met Ala Thr Lys Val Lys Phe Lys Tyr Lys Gly Glu Glu Lys Glu Val
1               5                   10                  15

Asp Ile Ser Lys Ile Lys Lys Val Trp Arg Val Gly Lys Met Ile Ser
            20                  25                  30

Phe Thr Tyr Asp Asp Asn Gly Lys Thr Gly Arg Gly Ala Val Ser Glu
        35                  40                  45

Lys Asp Ala Pro Lys Glu Leu Leu Glu Lys Leu Lys
    50                  55                  60

<210>  15
<211>  68
<212>  PRT
<213>  Artificial Sequence

<220>
<223>  Consensus sequence

<220>
<221>  VARIANT
<222>  (2)..(2)
<223>  Xaa is V, A or T

<220>
<221>  VARIANT
<222>  (3)..(3)
<223>  Xaa is T or K

<220>
<221>  VARIANT
<222>  (4)..(4)
<223>  Xaa is - or K

<220>
<221>  VARIANT
<222>  (6)..(6)
<223>  Xaa is R or K

<220>
<221>  VARIANT
<222>  (15)..(15)
<223>  Xaa is E or Q

<220>
<221>  VARIANT

<222>  (18)..(18)
<223>  Xaa is T or I

<220>
<221>  VARIANT
<222>  (31)..(31)
<223>  Xaa is V or I

<220>
<221>  VARIANT
<222>  (37)..(39)
<223>  Xaa Xaa Xaa is EGG or DN-

<220>
<221>  VARIANT
<222>  (57)..(57)
<223>  Xaa is M or L

<220>
<221>  VARIANT
<222>  (58)..(58)
<223>  Xaa is Q, D or E

<220>
<221>  VARIANT
<222>  (59)..(59)
<223>  Xaa is M or K

<220>
<221>  VARIANT
<222>  (61)..(67)
<223>  Xaa is -------, EKS--GK, EK---QK, ARAEREK, ARA-EKK, E-----K,
       EKS--G, ACAEREK oR ACA-EKK

<400>  15

Met Xaa Xaa Xaa Val Xaa Phe Lys Tyr Lys Gly Glu Glu Lys Xaa Val
1               5                   10                  15

Asp Xaa Ser Lys Ile Lys Lys Val Trp Arg Val Gly Lys Met Xaa Ser
            20                  25                  30

Phe Thr Tyr Asp Xaa Xaa Xaa Gly Lys Thr Gly Arg Gly Ala Val Ser
        35                  40                  45

Glu Lys Asp Ala Pro Lys Glu Leu Xaa Xaa Xaa Leu Xaa Xaa Xaa Xaa
    50                  55                  60

Xaa Xaa Xaa Lys
65

<210>  16
<211>  65
<212>  PRT
<213>  Artificial Sequence

<220>
<223>  Sequence N11 derived from Sac7d

<400>  16

Met Val Lys Val Lys Phe Val Met Phe Gly Lys Glu Lys Glu Val Asp
1               5                   10                  15

Thr Ser Lys Ile Val His Val Met Arg Gln Gly Lys Leu Val His Phe
            20                  25                  30

Trp Tyr Glu Asp Asn Gly Lys Ile Asp Lys Gly Phe Val Pro Glu Lys
        35                  40                  45

Asp Ala Pro Lys Glu Leu Leu Asp Met Leu Ala Arg Ala Glu Arg Glu
    50                  55                  60

Lys
65

<210>  17
<211>  65
<212>  PRT
<213>  Artificial Sequence

<220>
<223>  Sequence bindig to TNFa derived from N11

<220>
<221>  misc_feature
<222>  (7)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (21)..(22)
<223>  Xaa can be any naturally occurring amino acid

<220>
<221>  misc_feature
<222>  (24)..(24)
<223>  Xaa can be any naturally occurring amino acid

<220>
<221>  misc_feature
<222>  (26)..(26)
<223>  Xaa can be any naturally occurring amino acid

<220>
<221>  misc_feature
<222>  (29)..(29)
<223>  Xaa can be any naturally occurring amino acid

<220>

<221>  misc_feature
<222>  (35)..(35)
<223>  Xaa can be any naturally occurring amino acid

<220>
<221>  misc_feature
<222>  (41)..(41)
<223>  Xaa can be any naturally occurring amino acid

<220>
<221>  misc_feature
<222>  (44)..(44)
<223>  Xaa can be any naturally occurring amino acid

<220>
<221>  misc_feature
<222>  (46)..(46)
<223>  Xaa can be any naturally occurring amino acid

<400>  17

Met Val Lys Val Lys Phe Xaa Xaa Xaa Gly Xaa Glu Lys Glu Val Asp
1               5                   10                  15

Thr Ser Lys Ile Xaa Xaa Val Xaa Arg Xaa Gly Lys Xaa Val His Phe
            20                  25                  30

Trp Tyr Xaa Asp Asn Gly Lys Ile Xaa Lys Gly Xaa Val Xaa Glu Lys
        35                  40                  45

Asp Ala Pro Lys Glu Leu Leu Asp Met Leu Ala Arg Ala Glu Arg Glu
    50                  55                  60

Lys
65

<210>  18
<211>  65
<212>  PRT
<213>  Artificial Sequence

<220>
<223>  Sequence bindig to TNFa derived from N11

<220>
<221>  misc_feature
<222>  (7)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (21)..(21)

<223>  Xaa can be any naturally occurring amino acid

<220>
<221>  misc_feature
<222>  (26)..(26)
<223>  Xaa can be any naturally occurring amino acid

<220>
<221>  misc_feature
<222>  (29)..(29)
<223>  Xaa can be any naturally occurring amino acid

<220>
<221>  misc_feature
<222>  (35)..(35)
<223>  Xaa can be any naturally occurring amino acid

<220>
<221>  misc_feature
<222>  (41)..(41)
<223>  Xaa can be any naturally occurring amino acid

<220>
<221>  misc_feature
<222>  (44)..(44)
<223>  Xaa can be any naturally occurring amino acid

<220>
<221>  misc_feature
<222>  (46)..(46)
<223>  Xaa can be any naturally occurring amino acid

<400>  18

Met Val Lys Val Lys Phe Xaa Xaa Xaa Gly Xaa Glu Lys Glu Val Asp
1               5                   10                  15

Thr Ser Lys Ile Xaa His Val Met Arg Xaa Gly Lys Xaa Val His Phe
            20                  25                  30

Trp Tyr Xaa Asp Asn Gly Lys Ile Xaa Lys Gly Xaa Val Xaa Glu Lys
        35                  40                  45

Asp Ala Pro Lys Glu Leu Leu Asp Met Leu Ala Arg Ala Glu Arg Glu
    50                  55                  60

Lys
65